# EUROPEAN PATENT APPLICATION

(11) **EP 4 070 837 A1**
(43) Date of publication of application: **12.10.2022**
(21) Application number: 22167097.9
(22) Date of filing: 07.04.2022
(51) Int. Cl.: A61M 15/00

(54) **ELONGATE FORM MEDICAMENT CARRIER AND MEDICAMENT DISPENSER**

(30) Priority: 09.04.2021 GB 202105064
(71) Applicant: Merxin Ltd, King's Lynn PE30 5BY (GB)
(72) Inventor: Chapman, Alan, Chesterfield, S44 6TP (GB); Narshibhai, Sanjay, Birmingham, B27 6QD (GB)
(74) Representative: Basck Ltd

(57) **Abstract**

Disclosed is an elongate form medicament carrier (100) carrying multiple distinct medicament dose portions (108,110,112,114). The said carrier has a first end (102), a second end (104), a central portion (106) extending across the width of the elongate form medicament carrier, a first portion (116) between the first end and the central portion and containing a medicament active, or a mixture of medicament actives, and a second portion (118) between the second end and the central portion and containing a medicament active, or a mixture of medicament actives. The central portion of the carrier has a first end region (124), a unified length (132) and a second end region (126), the unified length has a trailing end (134) connecting to the first portion of the carrier via the first end region and connecting to the second portion of the carrier via the second end region, and the unified length has a leading end (136) connecting to a loop (138) of the central portion of the carrier.

## Description

### TECHNICAL FIELD

The present disclosure relates generally to a medicament carrier for administering medicament doses to patients; and more specifically, to elongate form medicament carriers for medicament dose portions and medicament dispensers with such medicament carriers.

### BACKGROUND

In recent past, developments in pharmaceutical and biomedical industries have led to improved formulation of new medicines and systems tailored for providing the formulated new medicines to patients. This is particularly the case for medicines delivered by inhalation. Diseases, for example, pulmonary disease (such as asthma and chronic obstructive pulmonary disease (COPD)), are managed primarily using maintenance therapy, symptomatic relief or combination therapy. For example, in the case of asthma, inhaled corticosteroids (ICS) are the cornerstone of maintenance therapy for symptomatic asthma patients, and ICS along with a long-acting beta2-adrenergic agonist (LABA) are generally prescribed. However, it is desirable to deliver a given formulation, alone or in combination with other medicament active ingredients, to patients with substantial bioavailability thereof and minimum patient reaction thereto.

Typically, a combination therapy comprises multiple medicament active components to be administered to the patients to offer added convenience to the patient. In this regard, the multiple medicament active components may be taken in combination or sequentially. However, certain medicament active components of the combination therapy are difficult to formulate and store as a single combined dosage, since such medicament active components may undergo undesired chemical interaction upon formulation and during storage. Different challenges are faced by the formulator for delivery of medicaments in dry powder form to the patients rather than as non-volatile solutions or from a pressurized metered dose inhaler.

Some dry powder medicament dispensers administer combination treatments by means of a plurality of elongate form carriers, such as blister strips. The plurality of carriers, along with the need for multiple sections in the dispensers for separate storage of the blister strips, results in added mechanical complexity and additional componentry in the medicament dispenser. These factors also contribute to an increase in the tendency for mechanical failure of such medicament dispensers. Moreover, multiple carriers contribute to additional space occupation, reduce device compactness, negatively impact ease of device assembly and increase the overall device cost. Furthermore, medicament dispensers that dispense from a pair of blister strips need to ensure that advancement of the individual blisters is coordinated with the corresponding blister in the other strip, starting from the first dosage to the end dosage therein.

Therefore, in light of the foregoing discussion, there exists a need to overcome the aforementioned drawbacks of the current medicament dispensers and provide an efficient, reliable, cost-effective and easy-to-use medicament dispenser.

### SUMMARY

The present disclosure seeks to provide an elongate form medicament carrier. The present disclosure also seeks to provide a medicament dispenser comprising a supply of medicament active consisting of one elongate form medicament carrier. The present disclosure seeks to provide a solution to the existing problem of complex conventional medicament carriers and dispensers and the high rate of failure associated therewith during progression of the medicament carriers in the medicament dispensers. An aim of the present disclosure is to provide a solution that overcomes at least partially the problems encountered in prior art, and that provides a reliable, effective, robust medicament carrier and dispenser for the delivery of multiple distinct medicament doses in a single combined dosage employing a single command action, while maintaining the integrity of the medicament carrier during progression thereof in the medicament dispenser.

In one aspect, an embodiment of the present disclosure provides an elongate form medicament carrier carrying multiple distinct medicament dose portions and having a first end, a second end, a central portion extending across the width of the elongate form medicament carrier, a first portion of the elongate form medicament carrier between the first end and the central portion and containing a medicament active, or a mixture of medicament actives, and a second portion of the elongate form medicament carrier between the second end and the central portion and containing a medicament active, or a mixture of medicament actives; wherein the central portion of the elongate form medicament carrier has a first end region, a unified length and a second end region, the unified length has a trailing end connecting to the first portion of the elongate form medicament carrier via the first end region and connecting to the second portion of the elongate form medicament carrier via the second end region, and the unified length has a leading end connecting to a loop of the central portion of the elongate form medicament carrier.

In another aspect, an embodiment of the present disclosure provides a medicament dispenser comprising a supply of medicament active, the supply of medicament active consisting of one elongate form medicament carrier according to the first aspect, said dispenser having a dispensing mechanism which is adapted to operate, upon each actuation of the medicament dispenser, to dispense medicament active from each of the first and second portions of the elongate form medicament carrier, said mechanism comprising,
a receiving station comprising first and second capstans that receive portions respectively of the first and second portions of the elongate form medicament carrier and at least one indexer that individually indexes the portions of each of the first and second portions of the elongate form medicament carrier,
the receiving station further comprising a manifold, for dispensing the medicament active from each of the first and second portions of the elongate form medicament carrier
the manifold having a first side corresponding to the first portion of the elongate form medicament carrier and a second side corresponding to the second portion of the elongate form medicament carrier,
the medicament dispenser further comprising a take-up spool that winds up the central portion of the elongate form medicament carrier; wherein the loop of the central portion of the elongate form medicament carrier is secured to a peg of the take-up spool.

Embodiments of the present disclosure substantially eliminate or at least partially address the aforementioned problems in the prior art, and enable delivery of multiple distinct medicament doses in a single combined dosage whilst reducing rucking of the elongate form medicament carrier and misfeeding of the elongate form medicament carrier around the take-up spool, thereby better controlling the tension in the medicament carrier and preventing the jamming of the medicament carrier in the vicinity of the manifold.

Additional aspects, advantages, features and objects of the present disclosure will be made apparent from the drawings and the detailed description of the illustrative embodiments construed in conjunction with the appended claims that follow.

It will be appreciated that features of the present disclosure are susceptible to being combined in various combinations without departing from the scope of the present disclosure as defined by the appended claims.

### BRIEF DESCRIPTION OF THE DRAWINGS

The summary above, as well as the following detailed description of illustrative embodiments, is better understood when read in conjunction with the appended drawings. For the purpose of illustrating the present disclosure, exemplary constructions of the disclosure are shown in the drawings. However, the present disclosure is not limited to specific methods and instrumentalities disclosed herein. Moreover, those skilled in the art will understand that the drawings are not to scale. Wherever possible, like elements have been indicated by identical numbers.

Embodiments of the present disclosure will now be described, by way of example only, with reference to the following diagrams wherein:
FIG. 1 is a perspective view of an elongate form medicament carrier, in accordance with an embodiment of the present disclosure;
FIGs. 2 and 3 are partial sectional views of a medicament dispenser, in accordance with an embodiment of the present disclosure;
FIG. 4 is a schematic partial illustration of an elongate form medicament carrier arranged in a medicament dispenser, in accordance with embodiments of the present disclosure; and
FIGs. 5A and 5B are diagrams of arrangements of a first capstan and a take-up spool, in accordance with two different embodiments of the present disclosure.

In the accompanying drawings, an underlined number is employed to represent an item over which the underlined number is positioned. A non-underlined number relates to an item identified by a line linking the non-underlined number to the item. When a number is non-underlined and accompanied by an associated arrow, the non-underlined number is used to identify a general item at which the arrow is pointing.

### DETAILED DESCRIPTION OF EMBODIMENTS

The following detailed description illustrates embodiments of the present disclosure and ways in which they can be implemented. Although some modes of carrying out the present disclosure have been disclosed, those skilled in the art will recognize that other embodiments for carrying out or practising the present disclosure are also possible.

The present disclosure provides an elongate form medicament carrier and a medicament dispenser comprising a supply of medicament active consisting of one elongate form medicament carrier. These may be employed in or as dry powder medicament dispensers for delivery of medicament to a patient by inhalation. The elongate form medicament carrier stores the medicament actives of the combination product to be administered to the patient separately and enables delivery of a combined medicament dose in response to a minimum number of patient actions. The elongate form medicament carrier and the medicament dispenser provide an innovative and cost-effective alternative to the conventional multiple elongate form carriers.

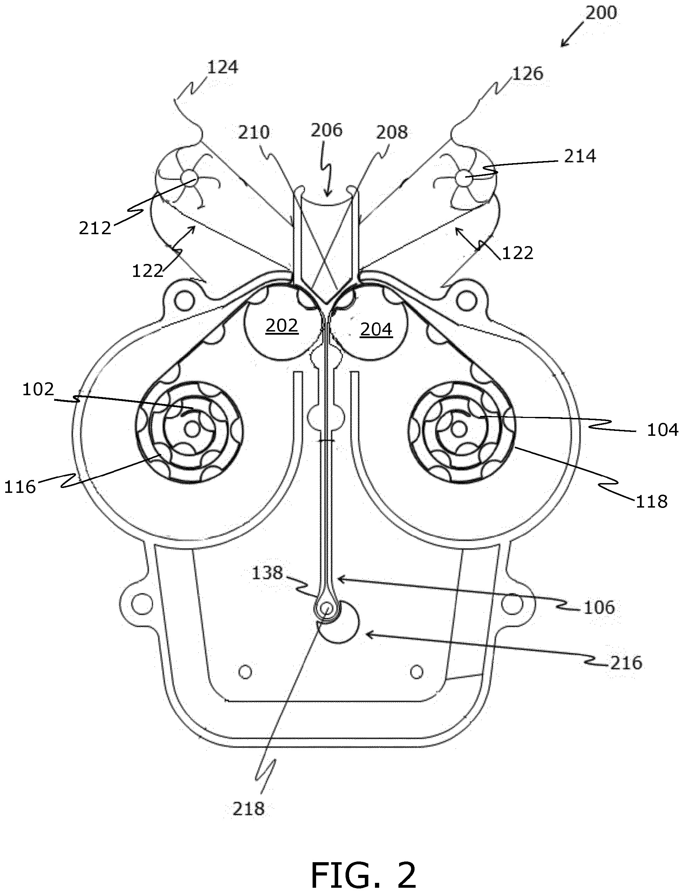

Notably, the single elongate form medicament carrier comprises an additional region, just below (in the orientation of Fig. 2) the closest of the pockets containing the multiple distinct medicament dose portions on different portions of the single elongate form medicament carrier. The additional region provides a junction with different portions of the single elongate form medicament carrier and a unified length that enables the different portions of the elongate form medicament carrier to advance through different parts of the medicament dispenser at the same rate, thereby eliminating a slack condition that can arise when two lengths of elongate are wound onto a single spool where the winding of one length is not coordinated with that of the other length due to differing winding rates.

Furthermore, employing a single medicament carrier carrying different medicament dose portions on different ends has benefits in manufacturing, filling and storage processes. The single elongate form medicament carrier reduces and/or simplifies the device componentry required in the medicament dispenser dispensing the medicament dose portions, thereby reducing complexity and overall cost of the medicament dispenser. Moreover, the single elongate form medicament carrier is assembled into the medicament dispenser with relatively greater ease, thereby simplifying the assembly of the medicament dispenser and reducing and/or simplifying the mechanisms required to drive the dispensing mechanism during use. Specifically, the single elongate form medicament carrier only requires a single take-up spool for winding of the used portions of the medicament carrier. Such reduced componentry reduces the risk of mechanical failure or jamming. Additionally, employing the single elongate form medicament carrier provides a single item of manufacture, reducing the amount of labelling and tracking of the medicament dose portions during manufacturing and assembly, and avoiding the need to pair them.

Pursuant to embodiments of the present disclosure, there is provided an elongate form medicament carrier carrying multiple distinct medicament dose portions. The term *"elongate form medicament carrier"* as used herein refers to a strip, fabricated from a thin sheet of flexible material or sheets of flexible materials, and bearing medicament. Such medicament carriers may be used to administer medicament dose portions (namely, medicament formulation or medicament product) to an intended user for example, a patient suffering from respiratory illness. Such medicament carriers can be employed to administer a plurality of medicaments useful for a variety of conditions such as asthma and pulmonary diseases such as COPD (chronic obstructive pulmonary disease). The medicament carrier is further operable to carry a plurality of distinct medicament dose portions and shaped in an elongated form so as to accommodate the distinct elements of the medicament carrier. The term *"medicament dose portion"* as used herein refers to a measured quantity of medicament that is prepared for dispensing to a patient. The administered dose is a whole number multiple of the medicament dose portion. Usually, the administered dose can be provided in one medicament dose portion.

The elongate form medicament carrier may be manufactured from a plastic material, an aluminium foil, a combination thereof, or any other suitable material, and may comprise recesses for forming pockets or blisters. Optionally, to store the medicament dose portions, multiple distinct regions (e.g., blisters or similar depressions) may be defined on the elongate form medicament carrier. The elongate form medicament carrier may comprise a pair of elongate sheets that have been attached together, wherein one of the pair of elongate sheets provides regions in which the medicament dose portions can be stored.

The elongate form medicament carrier has a first end, a second end, a central portion extending across the width of the elongate form medicament carrier, a first portion of the elongate form medicament carrier between the first end and the central portion and containing a medicament active, or a mixture of medicament actives, and a second portion of the elongate form medicament carrier between the second end and the central portion and containing a medicament active, or a mixture of medicament actives. It will be appreciated that the first portion and the second portion of the elongate form medicament carrier define two separate areas, and the first and second portions lie on either side of the central portion. Therefore, the first portion of the elongate form medicament carrier corresponds to the first end thereof. Similarly, the second portion of the elongate form medicament carrier corresponds to the second end thereof.

Throughout the present disclosure, the term *"medicament active"* refers to a pharmaceutical compound or a drug administered to a patient for healing, treating, altering, improving, restoring, relieving, and/or curing a particular condition, disease, or mental or physical state. Notably, a medicament active includes an active ingredient or a combination of active ingredients and inactive ingredients, optionally mixed with an excipient or dissolved in some other carrier for delivery to the patient. Furthermore, the mixture of medicament actives comprises a plurality of pharmaceutical compounds, wherein the plurality of pharmaceutical compounds do not react with each other.

The central portion of the elongate form medicament carrier usually does not comprise any regions to contain a medicament active, or a mixture of medicament actives.

The central portion of the elongate form medicament carrier has a first end region, a unified length and a second end region, the unified length has a trailing end connecting to the first portion of the elongate form medicament carrier via the first end region and connecting to the second portion of the elongate form medicament carrier via the second end region, and the unified length has a leading end connecting to a loop of the central portion of the elongate form medicament carrier. The central portion of the elongate form medicament carrier may be forked, to provide that the first end region is proximate to the first portion of the elongate form medicament carrier and the second end region is proximate to the second portion of the elongate form medicament carrier.

The term *"unified length"* as used herein refers to a segment of the central portion that starts immediately below (when orientated as shown in Fig. 2) the first and second end regions of the central portion and extends until the loop of the central portion. The unified length intervenes in the central portion to result in the loop and the end regions (i.e. the first and second end regions) that are proximate to the leading end and the trailing end of the unified length, respectively. The term *"loop"* as used herein refers to a "hair-pin loop like" structure achievable by folding of the central portion at the middle of the central portion. In such case, the loop brings the elongate form medicament carrier into a folded-configuration such that the lid elongate sheet corresponding to the first portion of the elongate form medicament carrier faces the lid elongate sheet corresponding to the second portion of the elongate form medicament carrier.

Optionally, the unified length is a bonded portion formed from two lengths of elongate form medicament carrier bonded together face-to-face. In this regard, the unified length consists of an extension of the first end region and an extension of the second end region, and the bond to unify those extensions is positioned as in the aforementioned folded configuration. The unified length is preferably permanently connected, such as by bonding, welding, and the like, to create such a bond. Beneficially, the single elongate form medicament carrier enables progression or advancement of the first and second portions of the elongate form medicament carrier at the same rate, when in operation. The unified length reduces the slack condition in the elongate form medicament carrier that results from any advancement of the base elongate sheet and the lid elongate sheet at different rates.

Optionally, the elongate form medicament carrier comprises a lid elongate sheet substantially secured to a base elongate sheet having pockets containing the multiple distinct medicament dose portions, and the base elongate sheet comprises the unified length of the central portion of the elongate form medicament carrier. The term *"base elongate sheet"* as used herein refers to one part of a pair of elongate sheets constituting the elongate form medicament carrier that comprises multiple distinct pockets (or recesses) formed therein to contain the medicament dose portions. The term *"lid elongate sheet"* as used herein refers to another part of the pair of elongate sheets constituting the elongate form medicament carrier that covers the multiple distinct pockets defined in the base elongate sheet. Optionally, the multiple distinct pockets are formed in the base elongate sheet, and the lid elongate sheet is hermetically sealed to the base elongate sheet. In such instance, the lid elongate sheet may be peeled, punctured, ruptured or torn to expose the medicament dose portion in the base elongate sheet to be administered to the patient. The sealing of the medicament dose portions implemented between the lid elongate sheet and the base elongate sheet ensures isolation of medicament dose portions from external factors such as humidity, dust particles, pathogens and so forth, and, if necessary, sterility of the medicament dose portions. It will be appreciated that both the first portion and the second portion comprise corresponding portions of a base elongate sheet and a lid elongate sheet each. And each of the base elongate sheets, i.e. the base elongate sheet corresponding to the first portion and the base elongate sheet corresponding to the second portion, comprises a plurality of pockets formed therein to contain the medicament dose portions. It will be appreciated that each of the base elongate sheet and the lid elongate sheet have a first end, a second end, and a central portion corresponding respectively to the first end, the second end, and the central portion of the elongate form medicament carrier. It will be appreciated that the lid elongate sheet may be similar in size to or slightly longer than the base elongate sheet.

The unified length of the central portion may be comprised in the base elongate sheet, specifically the central portion of the elongate sheet. The loop may also be comprised in the central portion of the base elongate sheet.

Optionally, the lid elongate sheet comprises at least the following successive layers: (a) paper; adhesively bonded to (b) polyester; adhesively bonded to (c) aluminium foil; that is coated with a heat seal lacquer for bonding to the base elongate sheet. Optionally, the base elongate sheet comprises at least the following successive layers: (a) oriented polyamide (OPA); adhesively bonded to (b) aluminium foil; adhesively bonded to (c) a third layer comprising a polymeric material. Notably, a multi-layered construction of the lid and base elongate sheets implemented using a combination of metal, paper and polymeric materials ensures a strong and reliable implementation of the lid and base elongate sheet and provides a superior barrier layer between the external contaminants and the medicament dose portions. Furthermore, the heat seal lacquer used to seal the lid elongate sheet to the base elongate sheet provides a sturdy hermetic sealing between the lid elongate sheet and the base elongate sheet. Optionally, the lid elongate sheet and the base elongate sheet may be bonded using bonding, welding, or any other method known to a person skilled in the art.

Optionally, the elongate form medicament carrier comprises a base elongate sheet as one of the pair of elongate sheets employed to form the elongate form medicament carrier, while the other elongate sheet employed to form the elongate form medicament carrier is a lid elongate sheet. Optionally, the lid elongate sheet has a central portion corresponding to the central portion of the elongate form medicament carrier, wherein the central portion of the lid elongate sheet is divided to provide a first inner end and a second inner end of the lid elongate sheet, and the first inner end and second inner end are not secured to the base elongate sheet. The base elongate sheet and the lid elongate sheet can be arranged in a split configuration or an unsplit configuration.

In the unsplit configuration the base elongate sheet and the lid elongate sheet are substantially secured (namely, sealed) together. In the unsplit configuration, the lid elongate sheet is not divided. In such case, when the elongate form medicament carrier is used in a medicament dispenser, the elongate form medicament carrier may be peeled, punctured, ruptured or torn to expose the medicament dose portion in the base elongate sheet to be administered to the patient.

In the split configuration the base elongate sheet and the lid elongate sheet are also substantially secured (namely, sealed) together. However, the first inner end and second inner end of the lid elongate sheet are not secured to the base elongate sheet. In this configuration the lid elongate sheet is divided, typically separating the lid elongate sheet into two lengths. This configuration is better adapted than the unsplit configuration for use in a medicament dispenser arranged for peeling the lid elongate sheet from the base elongate sheet.

In both configurations the lid elongate sheet of the elongate form medicament carrier typically extends from the first end to the second end of the elongate form medicament carrier, traversing the first and second portions thereof but not the unified length or the loop of the central portion. Typically, the lid elongate sheet covers all of the pockets in the base elongate sheet before the elongate form medicament carrier is used in a medicament dispenser.

In the split configuration, the central portion of the lid elongate sheet is divided, wherein the first inner end is proximate to the first portion of the lid elongate sheet and the second inner end is proximate to the second portion of the lid elongate sheet. The first inner end and the second inner end provide a structure similar to a lift-tab for peeling the lid elongate sheet from the base elongate sheet to expose the medicament dose portions. It will be appreciated that the first inner end of the lid elongate sheet is used to peel and expose the first set of medicament dose portions and similarly, the second inner end of the lid elongate sheet is used to peel and expose the second set of medicament dose portions. The lift-tabs provided may be harnessed to a dispensing mechanism, such as bobbins, to wind on the elongate form medicament carrier, specifically the lid elongate sheet.

Optionally, the medicament dose portions consist of a first set and a second set of medicament dose portions, the first set of medicament dose portions distributed in the first portion of the elongate form medicament carrier and the second set of medicament dose portions distributed in the second portion of the elongate form medicament carrier. In this regard, a first set and a second set of multiple distinct pockets are defined between the first and second portions of the base elongate sheet and the corresponding first and second portions of the lid elongate sheet for containing the first and second sets of medicament dose portions, respectively. Moreover, said first set of pockets is spaced along the length of the first portion of the elongate form medicament carrier and said second set of pockets is spaced along the length of the second portion of the elongate form medicament carrier. The first set of pockets may be distributed in the first portion of the elongate form medicament carrier. Similarly, the second set of pockets may be distributed in the second portion of the elongate form medicament carrier.

Optionally, the first portion of the elongate form medicament carrier is substantially the same size and shape as the second portion of the elongate form medicament carrier. Specifically, the elongate form medicament carrier is substantially symmetric across the central portion of the elongate form medicament carrier. It will be appreciated that the first portion of the elongate form medicament carrier (corresponding to the first set of medicament dose portions) has substantially the same length as the second portion of the elongate form medicament carrier (corresponding to the second set of medicament dose portions).

Optionally, the multiple distinct medicament dose portions of each of the first portion and the second portion of the elongate form medicament carrier are uniformly spaced. Notably, the distance between dose portions of the first set of multiple distinct pockets defined between the base elongate sheet and the lid elongate sheet is uniform. Similarly, the distance between dose portions of the second set of multiple distinct pockets defined between the base elongate sheet and the lid elongate sheet is uniform.

Optionally, the spacing of the multiple distinct medicament dose portions of the first portion of the elongate form medicament carrier is equivalent to the spacing of the multiple distinct medicament dose portions of the second portion of the elongate form medicament carrier. More optionally, the spacing between adjacent pockets of the first set of multiple distinct pockets defined between the base elongate sheet and lid elongate sheet is equal to the spacing between adjacent pockets of the second set of multiple distinct pockets. Since these spacings are equal and the length of the first portion of the elongate form medicament carrier is equal to the length of the second portion of the elongate form medicament carrier, it will be understood that the number (namely, count) of the first set of multiple distinct pockets is equal to the number of the second set of multiple distinct pockets.

Optionally, in contrast to the previous paragraph, the spacing of the multiple distinct medicament dose portions of the first portion of the elongate form medicament carrier is different from the spacing of the multiple distinct medicament dose portions of the second portion of the elongate form medicament carrier. Therefore, following the reasoning in the previous paragraph in contrasting fashion, in such embodiment, the number (namely, count) of the first set of multiple distinct pockets is different in comparison with the number of the second set of multiple distinct pockets.

Optionally, the length of the central portion is greater than the spacing of the multiple distinct medicament dose portions of both the first and the second portions of the elongate form medicament carrier. More optionally, the length of the central portion of the elongate form medicament carrier is significantly greater than the spacing between adjacent pockets in the first set of multiple distinct pockets and in the second set of multiple distinct pockets.

Optionally, the dose portions in the second set of medicament dose portions are different from the dose portions in the first set of medicament dose portions. In an embodiment, the dose portions in the second set of medicament dose portions differ from the dose portions in the first set of medicament dose portions in that the dose portions in the second set of medicament dose portions comprise a different amount (namely, a higher amount or a lower amount) of the medicament active (or mixture of medicament actives) than the dose portions in the first set of medicament dose portions. In another embodiment, the dose portions in the second set of medicament dose portions differ from the dose portions in the first set of medicament dose portions in that the dose portions in the second set of medicament dose portions comprise a different frequency of administering of the medicament active (or mixture of medicament actives) to the patient than the frequency of administering of the dose portions in the first set of medicament dose portions. It will be appreciated that the doses portions in the first and second sets of medicament dose portions is likely to depend on at least one of: the patient's age, symptoms, severity of condition, a prescribed medication, and medication side effects.

Optionally, the dose portions in the second set of medicament dose portions differ from the dose portions in the first set of medicament dose portions in that the dose portions in the second set of medicament dose portions contain a medicament active, or a mixture of medicament actives, that is different from that in the first set of medicament dose portions. In an example, the first set of medicament dose portions contains a medicament active, or a mixture of medicament actives, that is an anti-inflammatory drug (such as a corticosteroid, or any other steroid), the second set of medicament dose portions contains a medicament active, or a mixture of medicament actives, that is a bronchodilator (such as a long-acting beta agonist (LABA) or a long-acting muscarinic receptor agonist (LAMA)). Notably, the bronchodilator as the active medicament component is employed for dilating bronchi and bronchioles of the patient to whom it is administered, and for increasing airflow to the lungs. Furthermore, the anti-inflammatory as the active medicament component in the second set of medicament dose portions is employed for reducing swelling in the patient, decreasing airway sensitivity caused by inflammation and reducing mucus production. A combination of medicament dose portion comprising bronchodilator and anti-inflammatory is generally administered to a patient suffering from asthma. It will be appreciated that in combination therapy the different medicament actives typically function synergistically to improve a severity of a given medical condition, such as asthma in the above example, over a period of time of using the said medicament actives.

Optionally, the medicament dose portions are in powdered, liquid, solid or vaporised form. Optionally, each medicament dose portion of each portion of the elongate form medicament carrier comprises a single active medicament component. Alternatively, each medicament dose portion of each portion of the elongate form medicament carrier comprises a plurality (i.e. more than one) of active medicament components. Notably, in such case, the first set of medicament dose portions may comprise a mixture of medicament actives comprising plural active medicament components. Also, the second set of medicament dose portions may comprise a mixture of medicament actives comprising plural active medicament components, wherein at least one active medicament component in the second set of medicament dose portions is different from the plural active medicament components in the first set of medicament dose portions.

The person skilled in the art will recognize many variations, alternatives, and modifications of embodiments of the present disclosure, such as use of a single-strip elongate form medicament carrier by bonding, in hair-pin loop turn, an end of the said single-strip elongate form medicament carrier not containing the medicament dose portions (similar to the central portion of the disclosed elongate form medicament carrier) upon itself to result in an additional region (similar to the central portion of the disclosed elongate form medicament carrier) for reliably collecting the used single-strip elongate form medicament carrier. For example, a second loop may be formed to provide a second engagement for the take-up spool of the medicament dispenser.

Optionally, the length of the unified length is in the range 5 millimetres to 30 millimetres. The length of the unified length may be for example from 5, 10, 15, 20 or 25 millimetres (mm) up to 10, 15, 20, 25 or 30 mm.

Pursuant to embodiments of the present disclosure, there is provided a medicament dispenser comprising a supply of drug, the supply of drug consisting of one elongate form medicament carrier as described herein above. The medicament dispenser has a dispensing mechanism which is adapted to operate, upon each actuation of the medicament dispenser, to dispense medicament active from each of the first and second portions of the elongate form medicament carrier. Specifically, the medicament dispenser is employed by a patient to administer a single distinct medicament dose portion from each of first and second sets of medicament dose portions stored in the first and second portions respectively of the elongate form medicament carrier, wherein the patient employs the dispensing mechanism to cause the actuation of the medicament dispenser. It will be appreciated that a single distinct medicament dose portion from each of the first and second sets of medicament dose portions is administered as a mixture of the medicament active (or mixture of medicament actives) in the first medicament dose portion and the medicament active (or a mixture of medicament actives) in the second medicament dose portion.

In regard to the medicament dispenser described above comprising the dispensing mechanism, the dispensing mechanism comprises a receiving station comprising a first and second capstan that receive portions respectively of the first and second portions of the elongate form medicament carrier. The receiving station receives portions of the elongate form medicament carrier. Specifically, the receiving station comprises the first capstan and the second capstan to receive portions, i.e. the first portion and the second portion respectively, of the elongate form medicament carrier. The dose portions may be stored in pockets formed in a base elongate sheet and protected (namely, covered) by a lid elongate sheet. The receiving station may comprise a recess in each of the first and second capstans that registers (or positions) the pocket adjacent to a manifold. Specifically, the first and second capstans comprise recesses for accommodating pockets of the elongate form medicament carrier and the capstans each position a given pocket adjacent to the manifold for delivery of the dose portion therefrom. Alternatively, a dose portion may be adhered to a surface of the elongate form medicament carrier in a region that can be encompassed by the manifold, and the receiving station comprises the first and second capstans configured to bring the dose portion into registration with the manifold, ideally directly adjacent to the manifold. Optionally then, the dose portion may be protected by a lid elongate sheet in the form of a liner, i.e. not strongly adhered to the base elongate sheet, but readily peeled therefrom.

Optionally, the receiving station may be implemented as a single receiving station that receives each of the first and second portions of the elongate form medicament carrier. Alternatively, optionally, the receiving station may be implemented as plural distinct receiving stations for receiving respective portions of first and second portions of the elongate form medicament carrier. In such case, each of the plural distinct receiving stations comprises a capstan, such as the first capstan and the second capstan, wherein the first capstan receives the first portion of the elongate form medicament carrier and the second capstan receives the second portion of the elongate form medicament carrier. The capstans are in the form of dimpled cylinders, and pockets of the elongate form medicament carrier may be carried in the dimples upon rotation of the capstan in order to advance a dose portion.

Optionally, the dispensing mechanism comprises the release for releasing a distinct medicament dose portion from each of the portions of the elongate form medicament carrier respectively carrying each of the first and second sets of medicament dose portions. Notably, the release enables release of each of the first and second sets of medicament dose portions into the manifold, so that it can be administered to the patient. It will be appreciated that the release is triggered by an actuation means on the medicament dispenser, wherein the patient uses the actuation means to cause actuation of the medicament dispenser and to release each time one of the first and one of the second sets of medicament portions. In some embodiments, the actuation means is implemented as the mouthpiece cover or as a button, wherein the patient uncovers the mouthpiece of the dispenser or presses the button to trigger the release. In another embodiment, the actuation means is implemented as a slider, wherein the patient slides the slider to trigger the release.

Optionally, said release comprises means to access medicament carried by said elongate form medicament carrier by a rupturing, puncturing, tearing or peeling action. Specifically, as aforementioned the elongate form medicament carrier comprises first and second sets of medicament dose portions, wherein each single distinct medicament dose portion is sealed thereon. Therefore, to release a medicament portion from the elongate form medicament carrier, an action such as rupturing, puncturing, tearing or peeling needs to be performed depending upon the type of the elongate form medicament carrier and the type of medicament dispenser. Such action by the release discharges the medicament doses carried by the elongate form medicament carrier enabling the administration of the medicament to the patient. The released dose may temporarily be retained on the elongate form medicament carrier until dispensed therefrom. Thus, the release may occur before the dose is fully registered by the at least one receiving station.

In an example, the elongate form medicament carrier is implemented as a peelable blister strip form medicament carrier and the release comprises a peeler for peeling apart the peelable blister strip form medicament carrier. In an instance when the release provides the peeling action, the receiving station may comprise a stationary blade that is positioned between already separated portions of the base elongate sheet and the lid elongate sheet. As the elongate form medicament carrier is wound on, the blade progressively separates further portions of the lid elongate sheet from the base elongate sheet by providing diverging paths either side of the blade. Optionally, the medicament dispenser comprises a guide for guiding the base elongate sheet and lid elongate sheet along separate paths subsequent to their peeling apart. Notably, the guide is employed to guide the lid elongate sheets towards the said pair of bobbins and the base elongate sheet towards the take-up spool.

In another example, the release provides a puncturing action and the receiving station may comprise a revolving punch. Such a punch may puncture a hole in the lid elongate sheet in line with a dose portion before that dose portion is registered with the manifold. Alternatively, the dose portion may be registered with the manifold, and subsequently the punch may provide a through hole piercing the base elongate sheet and the lid elongate sheet to provide a through passage. The dose may then be delivered in an airstream, which is typically provided when the patient inhales through the medicament dispenser, through the through passage.

The dispensing mechanism of the medicament dispenser further comprises at least one indexer for individually indexing the distinct medicament dose portions of each of the first and second portions of the elongate form medicament carrier respectively carrying each of the first and second sets of medicament dose portions. The receiving station may be associated with at least one indexer that individually indexes the portions of each of the first and second portions of the elongate form medicament carrier. The indexer may comprise an arrangement of gears driven by a predefined extent of travel per medicament dose. Notably, the arrangement of gears is configured to register the medicament dose(s) with the manifold of the receiving station, to align dose portions with the airway openings, i.e. to align individual dose portions with corresponding openings in the manifold. In an implementation of the medicament dispenser, the at least one indexer employs a mechanical counter, visible to the user, typically comprising a series of disks mounted on an axle, with the digits zero through nine marked on edges of the disks, to keep count of the medicament dose portions released from the elongate form medicament carrier. Ideally, the at least one indexer is configured on the medicament dispenser to provide a count of the number of medicament dose portions from the first and second sets of medicament dose portions that have been administered to the patient (or, have been released from the medicament dispenser). Herein, the at least one indexer is coupled to the aforementioned release, wherein the at least one indexer indexes a change in count of administered medicament dose portions as the medicament dose portions are released by the release. The at least one indexer enables a patient to (a) keep a count of the number of medicament dose portions that have been administered to them or alternatively that remain to be administered to them, and/or (b) determine whether the medicament dose portions have been released from the elongate form medicament carrier and are ready to be taken up, as the number on the at least one indexer changes as the medicament dose portions have been released by the release. Alternatively, optionally, the at least one indexer is implemented as a digital counter, for example as an electronic counter.

Optionally, a single indexer indexes the first and second sets of medicament dose portions. Specifically, a single indexer is employed in the medicament dispenser in an instance wherein the first and second sets of medicament dose portions are always to be released and administered to the patient simultaneously. It will be appreciated that since the first and second sets of medicament portions are released from the elongate form medicament carrier simultaneously every time, the number of administered medicament dose portions from the first set and second set will always be the same. Therefore, a single indexer is employed to index release of the first and second sets of the medicament dose portions.

Optionally, plural distinct indexers index the first and second sets of medicament dose portions. Specifically, plural (namely, a pair of) distinct indexers are employed in an instance when the first and second sets of medicament dose portions are not always to be released simultaneously. In an instance, one medicament dose portion from the second set of the medicament dose portions is administered for every two medicament dose portions administered from the first set of medicament dose portions. In other words, a medicament dose portion from the second set of medicament dose portions is released for every alternate medicament dose portion released from the first set of medicament dose portions. Therefore, since the count of medicament dose portions released from the first and second set of medicament dose portions is different, distinct indexers are employed to index the first and second sets of medicament dose portions. Optionally, said plural distinct indexers are mutually coupled. Notably, the plural distinct indexers are coupled such that the count shown by the indexers is in tandem with the medicament dose portions released from the first set and second set of medicament dose portions.

The receiving station further comprises a manifold, for dispensing the medicament active from each of the first and second portions of the elongate form medicament carrier. The manifold is positioned to be in communication with the distinct medicament dose portions releasable by said release to enable their dispensing to the patient. Specifically, upon release, the distinct medicament dose portion from each of the first and second portions of the elongate form medicament carrier is received by the manifold, wherein the patient uses the manifold of the medicament dispenser to receive the medicament dose portions. In an example, the patient may apply suction to the medicament dose portions through the manifold.

Optionally, the manifold provides access between a compartment of the medicament dispenser housing the elongate form medicament carrier and a chamber for mixing a dispensed dose with an airstream for delivery to a patient. Specifically, when the patient applies a suction to a mouthpiece or a nose piece of the medicament dispenser, due to the change in pressure an airstream (namely, an airflow) is introduced into the medicament dispenser. Such airstream may carry the medicament dose portions from the manifold (or from the elongate form medicament carrier) to the patient. Advantageously, when the manifold is positioned to be in communication with a distinct dose portion, the elongate form medicament carrier is practically airtight with the manifold around the opening of a pocket containing that dose portion, in order to control airflow through the medicament dose portion to dispense it.

The manifold has a first side corresponding to the first portion of the elongate form medicament carrier and a second side corresponding to the second portion of the elongate form medicament carrier. Optionally, a single common manifold passage is provided, which communicates with the distinct medicament dose portions via respective sides of the manifold. Specifically, the first side of the of the manifold receives the first set of medicament dose portions from the first portion of the elongate form medicament carrier. Similarly, the second side of the of the manifold receives the second set of medicament dose portions from the second portion of the elongate form medicament carrier. Notably, a single distinct medicament portion from each of the first set and second set of medicament dose portions is received into the single common manifold passage, whereby the patient receives a mixture of each one of the first set and second set of medicament dose portions via a mouthpiece or nosepiece. Optionally, said common manifold passage is shaped to encourage mixing of said released medicament dose portions. Specifically, openings in the manifolds that are in communication with respective medicament dose portions are angled so that the airflows from the respective pockets converge, which encourages the single distinct medicament dose portions from each of the first set and second set of medicament dose portions to mix with each other in the single common manifold passage.

In an exemplary embodiment, in which the dose portions are in pockets, a pocket of the base elongate sheet is registered sealingly adjacent a manifold. Herein, the manifold is divided into an inlet portion and an outlet portion. The outlet portion is configured in communication with a mouthpiece or nosepiece of the medicament dispenser via an outlet passage, and the inlet portion is configured in communication with an external inlet via an inlet passage that allows air to be drawn into the device due to suction applied by the patient on the mouthpiece or nosepiece. Optionally, a by-pass airflow may be provided linking the inlet passage with the outlet passage, so that some of the airstream by-passes the pocket. In use, the airstream from the inlet passage enters the pocket through the inlet portion of the manifold. Consequently, the airstream scours the contents (the dose) of the pocket, hence dispensing the dose, and carries it via the outlet portion of the manifold, along the outlet passage to the patient.

Alternatively, optionally, there are two manifolds, one for each of the first and second sets of medicament dose portions.

The medicament dispenser further comprises a take-up spool that winds up the central portion of the elongate form medicament carrier. Specifically, the take-up spool is rotated and starts to wind up the central portion of the elongate form medicament carrier and then proceeds to wind the first and second portions of the elongate form medicament carrier onto the take-up spool after doses have been dispensed from the first and second sets of medicament portions carried therein respectively. In an instance, the lid elongate sheet is punctured to expose the medicament dose portions carried by the elongate form medicament carrier in the first and second portion thereof for administration. Herein, after the medicament dose portions have been delivered from the elongate form medicament carrier, the elongate form medicament carrier along with the punctured lid elongate sheet is wound onto the take-up spool. In another instance, the base elongate sheet and the lid elongate sheet are peeled apart by the release (as described herein above) to dispense the medicament portions carried by the elongate form medicament carrier in the first and second portion thereof. Therefore, the take-up spool rotates and winds the base elongate sheet as the lid elongate sheet is peeled off therefrom. The mechanism of the take-up spool is triggered by the actuation means triggering the release. Specifically, the actuating means (such as a button or slider) may trigger the rotation of the take-up spool such that at least one of the pair of the elongate sheets employed to form the elongate form medicament carrier is wound up around the take-up spool. Optionally, the rotation motion of the take-up spool enables the peeling apart of the lid elongate sheet from the base elongate sheet. Specifically, a spooling mechanism of the take-up spool causes the elongate sheet to coil in on itself as further portions of the elongate sheets are fed through the capstans towards the take-up spool.

Optionally, the radius of the take-up spool, where the central portion of the elongate form medicament carrier is initially wound on, is less than the radii of the first and second capstans at the respective points where the elongate form medicament carrier leaves the first and second capstans. The radii of the take-up spool and the first and second capstans determine the lengths of the elongate form medicament carrier that is wound on the corresponding take-up spool and the first and second capstans. Notably, reducing the radius of the take-up spool reduces the length of the elongate form medicament carrier, i.e. the central portion, wound thereon. Beneficially, by reducing the radius of the take-up spool, the previously mentioned slack condition in the elongate form medicament carrier can be reduced as the differences between the first portion and the second portion of the base elongate sheets' running radii (namely, on the take-up spool) are reduced. The term *"running radius"* refers to the radius of the portion of the elongate form medicament carrier on the capstans or the take-up spool of the medicament dispenser.

In an instance, where the base elongate sheet and the lid elongate sheet are peeled off to expose the medicament dose portions, the additional region of elongate form medicament carrier connection enables reduction in jamming of the elongate form medicament carrier's base elongate sheet on the first and second capstans of the medicament dispenser as the lid elongate sheet winds onto the first and second bobbins of the medicament dispenser. Due to the said additional region, the first portion of the base elongate sheet and the second portion of the base elongate sheet travel at the same rate around the capstan, which eliminates slackness occurring in one of the portions of the base elongate sheet. Surprisingly, this initial coordination of the feed of the first and second portions of the base elongate sheet and subsequent uptake onto the take-up spool is sufficient to ensure that slackness continues to be eliminated for the remainder of the doses dispensed from the elongate form medicament carrier. Beneficially, simultaneous advancement of the different parts of the elongate form medicament carrier results in dispensing medicament doses without any slackness in the elongate form medicament carrier. It will be appreciated that in case of slackness in the elongate form medicament carrier, as tends to be experienced in carriers of the prior art, the elongate form medicament carrier tends to lift off (or raises) at the point where it enters the manifold for dispensing the medicament doses. Such a raised portion can result in a mechanical failure of the first and/or second capstans (as well as the medicament dispenser itself) to push the said raised portion through the manifold, thereby increasing the torque significantly and/or resulting in the elongate form medicament carrier jamming. By providing an elongate form medicament carrier in accordance with the disclosure herein, it makes both the resultant elongate sheets (strips) advance at the same rate, e.g. for the first eight or so doses, and thus eliminates the slack condition. Thereafter, as the strip has already been wound onto the take-up spool of the medicament dispenser somewhat, there is the ability for the portion of the elongate sheet already on the take-up spool to 'give a little' and also take up most of the slack thus overcoming the jamming condition.

Moreover, the loop of the central portion of the elongate form medicament carrier is secured to a peg of the take-up spool. Notably, the central portion of the elongate form medicament carrier is secured to the take-up spool, wherein the elongate form medicament carrier continues to wind around the take-up spool starting with the central portion of the elongate form medicament carrier. Specifically, the central portion of the elongate form medicament carrier is secured to the take-up spool by a peg. Specifically, the peg is implemented as a short pin or bolt, around which the central portion of the elongate form medicament carrier is secured. The peg is typically positioned parallel to the rotational axis of the take-up spool.

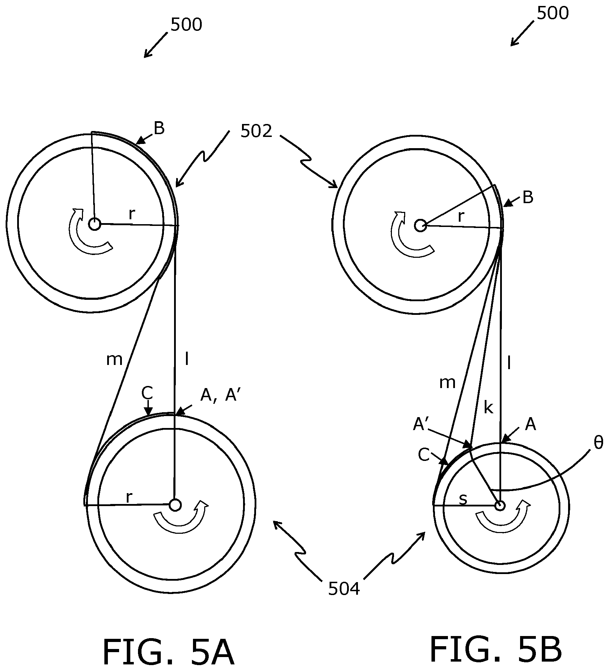

Optionally, the take-up spool has an axis of rotation such that the edge of the take-up spool facing the manifold rotates towards the first side of the manifold, i.e. towards that side of the whole device relative to the manifold; wherein the peg is positioned away from the axis of rotation and in a rotationally advanced position, relative to a datum line from the point where the elongate form medicament carrier leaves the first capstan to the axis of rotation of the take-up spool, prior to the indexer being driven for the first time after assembly of the elongate form medicament carrier into the medicament dispenser. As discussed above, the indexer may comprise an arrangement of gears driven by a predefined extent of travel per medicament dose. Notably, the arrangement of gears is configured to register the exit of a specific length of the elongate sheets from the receiving station. As the arrangement of gears advances, the take up spool is driven to receive the same specific length of the central portion of the elongate medicament carrier. This is achieved taking into account the respective radii of the capstans and the take up spool. The rotation of the take up spool rotates the position of the peg from its already rotationally advanced position to a further rotationally advanced position. The elongate form medicament carrier winds onto the take-up spool as it rotates in a direction towards the first side of the manifold, i.e., towards the side of the medicament dispenser that houses the first portion of the elongate medicament carrier. Notably, the direction of winding of the elongate form medicament carrier on the take-up spool is opposite to the direction of unwinding of the elongate form medicament carrier from the first capstan, as indicated by the hollow curved arrows in FIGS. 5A and 5B. It will be appreciated that the elongate form medicament carrier is bonded to result in the additional region, therefore the first and second portions of the elongate form medicament carrier wind on the take-up spool in a single direction. Specifically, the peg is positioned away from the axis of rotation and rotates by exerting a force on the central portion of the elongate form medicament carrier looped there around to enable winding of the said elongate form medicament carrier on the take-up spool. The term *"datum line"* refers to a point of reference from the point where the elongate form medicament carrier leaves the first capstan to the axis of rotation of the take-up spool. The datum line allows the angle of the rotationally advanced position of the take-up spool to be defined.

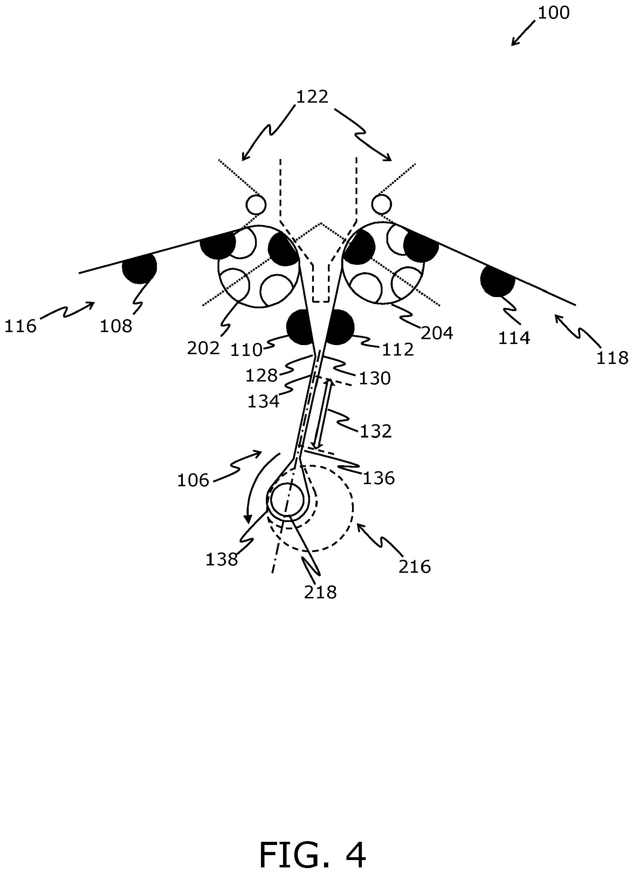

Optionally, the peg is in a position rotationally advanced at least 30 degrees relative to the datum line. The position of the peg is for example from 30, 40, 50, 60, 70 or 80 degrees up to 40, 50, 60, 70, 80 or 90 degrees. Optionally, the peg is in a position rotationally advanced at least 60 degrees relative to the datum line. The position of the peg is for example from 60, 70 or 80 degrees up to 70, 80 or 90 degrees. The position of the peg is for example 75 degrees. Optionally, the peg is in a position rotationally advanced not more than 90 degrees relative to the datum line. Notably, at 90 degrees of peg rotational advancement, the elongate form medicament carrier wrapping position angle becomes zero, i.e. the elongate form medicament carrier arrives tangentially at the take-up spool, so greater angles make no difference. Beneficially, the rotation of the peg, starting from an initial position in the said range of rotational advancement angles, enables effective winding of the elongate form medicament carrier onto the take-up spool in a single, correct direction after the medicament dose portions have been delivered from the elongate form medicament carrier. For example, providing a 10 o'clock start position of the loop location post (i.e. peg), as shown in Fig. 4, rather than a 12 o'clock one, results in (a) pulling the elongate form medicament carrier away from the capstan to a greater extent during the initial winding, and (b) reducing the slack condition in the elongate form medicament strip to enable the medicament carrier and the medicament dispenser to work more reliably. Additionally, as discussed above, the additional region of the elongate form medicament carrier assists in efficient winding of the elongate form medicament carrier on the take-up spool by maintaining the desired tension in the elongate form medicament carrier.

The more advanced starting position leads to less spooling slack and the possibility that excessively wound elongate would feed over the far side (as would be indicated by angles less than zero) of the take-up spool is also eliminated. In other words, by changing the location post of the peg of the take-up spool, the excess length of the elongate form medicament carrier is diminished non-linearly to zero as θ approaches 90 degrees; therefore a 90 degree position is reached by the peg with a shorter feed or wrap. Notably, from angles in excess of approximately 90 degrees, the elongate form medicament carrier wrapping position no longer changes with angle. With further wrapping, the position on the take-up spool at which wrapping of the elongate form medicament carrier occurs is about the 90 degree position and the calculated spooling slack becomes zero, so greater angles make no difference.

Optionally, the indexing mechanism is configured to drive the capstans and the take-up spool with gears such that their respective gear ratios are in inverse proportion to their respective radii. Notably, when the radius of the take up spool is less than the radius of the capstans, the 90 degree position is reached with a relatively short degree of winding, compared to the situation for equal radii, and consequently the aforementioned slack condition is reduced. As discussed above, the indexer comprises an arrangement of gears driven by a predefined extent of travel per medicament dose, in order to align the medicament dose with the opening in the manifold. Notably, the arrangement of gears is configured to bring about registration of the medicament dose(s) for dispensing, i.e. from the corresponding first and second capstans. In this regard, a first gear ratio corresponding to the first capstan drives the first capstan to rotate in a direction opposite to the direction of rotation of the second capstan driven by a corresponding second gear ratio. The rotational motion of the capstans results in a translational motion of the elongate form medicament carrier running thereon. The term "gear ratio" as used herein refers to two or more meshing gears working in a sequence to elicit a desired motion as a function of the driving and the driven gear. In an example, a gear ratio of 1:1 results from pairing two similar gears (such as bevel gears) with the same number of teeth, such that the number of teeth on the driven gear is the same as the number of teeth on the driving gear. Specifically, relative respective gear ratios of the take-up spool and the capstan may be based upon the relative respective running radii. Moreover, the gear ratio may be determined based on a size or radius of the corresponding capstans or take-up spool, wherein the size of the gear ratio of the take-up spool relative to that of the capstan is greater than 1. It may be advantageous for the gear ratio to be adjusted slightly downwards to avoid over-tension towards the end of use of the elongate form medicament carrier. The consequent introduction of slack towards the start of use will be offset by the provisions made in this disclosure.

In an exemplary implementation, the gearing arrangement comprises a plurality of gears driven through a lid driver gear. The lid driver gear is coupled with a plurality of capstan gears, specifically a first capstan gear mutually coupled to a second capstan gear, wherein the first capstan gear drives the first capstan and the second capstan gear drives the second capstan. Furthermore, the lid driver gear is coupled to a take-up spool gear, wherein the take-up spool gear drives the take-up spool using a connecting gear. Moreover, the first capstan gear is coupled to a first bobbin gear (driving the first bobbin) and the second capstan gear is coupled to a second bobbin gear (driving the second bobbin). Herein the dispensing mechanism comprises a lid driver connected to the lid driver gear, wherein the first capstan gear and the second capstan gear are driven by the lid driver which coordinates its rotation with the rotation of the take-up spool gear and with the first bobbin gear and the second bobbin gear.

Optionally, the medicament dispenser comprises grip elements configured to flatten the elongate form medicament carrier after dispensing the medicament doses therefrom. Specifically, the grip elements operate in a manner similar to a mangle, wherein the grip elements squeeze the used elongate sheets therethrough together. Alternatively, the grip elements act against a plate and squeeze each elongate sheet through either side of the plate. Specifically, the grip elements compress the regions for storing medicament dose portions in the elongate form medicament carrier. Moreover, during insertion of the elongate form medicament carrier into the medicament dispenser, the elongate form medicament carrier is initially assembled into the medicament dispenser by securing part of the central portion thereof around a peg and the additional region of the central portion proximate to the grip elements.

Optionally, the medicament dispenser comprises a pair of bobbins comprising a first bobbin and a second bobbin, the pair of bobbins configured to wind the lid elongate sheet of the elongate form medicament carrier onto the pair of bobbins, starting with the first end region of the lid elongate sheet on the first bobbin and the second end region of the lid elongate sheet on the second bobbin, and continuing to wind first and second portions of the lid elongate sheet onto the respective first and second bobbins after dispensing doses from the first and second sets of medicament portions respectively. In particular, the first end region of the lid elongate sheet is coupled to the first bobbin, and the second end region of the lid elongate sheet is coupled to the second bobbin, such that after a medicament portion from each of the first and second sets of medicament portions has been dispensed, those portions of the lid elongate sheet that have been peeled apart from the base elongate sheet during the dispensing are wound around the pair of bobbins. Herein, the pair of bobbins are capable of rotational motion to aid winding of the lid elongate sheet.

In an implementation of the present disclosure, the medicament dispenser comprises the take-up spool and the pair of bobbins employed to wind up the base elongate sheet and lid elongate sheet, respectively. In such implementation, the first bobbin and the second bobbin are driven by a mechanism which coordinates their rotation with the rotation of the take-up spool. Specifically, the mechanism coordinating the rotation ensures the coordinated rotation of the take-up spool and the pair of bobbins such that for every actuation of the medicament dispenser, a single distinct medicament dose portion from each of the first and second sets of medicament dose portions is dispensed by peeling apart the lid elongate sheet and the base elongate sheet.

It will be appreciated that the medicament dispenser comprises the actuating means (such as the mouthpiece cover, a button or slider) that the patient employs to release the medicament dose portions from the elongate form medicament carrier. The actuating means is operable to trigger the dispensing mechanism, and specifically, the coordinated rotation of the take-up spool and the pair of bobbins, wherein upon rotation, the take-up spool winds the base elongate sheet and the pair of bobbins wind the lid elongate sheet. Consequently, due to such winding, the base elongate sheet and lid elongate sheet are peeled apart to release one medicament dose portion of each of the first and second sets of medicament dose portions into the manifold. Subsequently, the patient may use suction to receive the medicament dose portions, thereby effecting administering thereof.

Optionally, an elongate form medicament carrier may be inserted into a medicament dispenser of the disclosure during manufacture. Optionally, the elongate form medicament carrier is assembled into a cartridge (or cassette). The cartridge is designed to insert into a body of the medicament dispenser. The cartridge may comprise the feed spools, the capstan(s), the take-up spool, and the bobbin(s). In an exemplary process, the first portion of the elongate form medicament carrier is wound onto a first feed spool and the second portion of the elongate form medicament carrier is wound onto a second feed spool. The first end region of the lid elongate sheet is lifted and attached to an axle of the first bobbin and the second end region of the lid elongate sheet is lifted and attached to an axle of the second bobbin. Such a process may be carried out on a carriage with spindles that allows the feed spools and bobbins to be positioned for transfer to a unit of the medicament dispenser. The central portion of the elongate form medicament carrier is dragged by a moving spindle to a position corresponding to the take-up spool. As the feed spools and bobbins are brought up to the unit, the moving spindle feeds a short section, i.e. the loop, of the central portion onto a peg of the take-up spool, and other spindles arrange for the other parts of the elongate form medicament carrier to be fed onto the receiving station capstans.

Optionally, any or all components of the dispensing mechanism are drivable by an electronic drive system. Specifically, the medicament dispenser may comprise an electronic drive system housed therein coupled with a power source (such as a battery), wherein the electronic drive system may drive the components such as release and optionally, may further drive the rotation of the components such as the capstans, take-up spool and the pair of bobbins.

Optionally, the medicament dispenser additionally comprises an electronic data management system. Optionally, such an electronic data management system comprises control instructions for the electronic drive system. Furthermore, optionally, the electronic data management system may store a count of the administered medicament dose portions, details of the medicament actives in the first and second sets of medicament dose portions, and timings of when the medicament dose portions are to be administered.

Optionally, the medicament dispenser is in a reloadable form. Specifically, the medicament dispenser is configured in a manner such that a second elongate form medicament carrier can be loaded into the medicament dispenser once the existing elongate form medicament carrier has been expended and has been removed.

Optionally, the medicament dispenser comprises a body; a holder, shaped to fit within said body and movable relative to said body; and receivable by said holder, a reload cassette containing one elongate form medicament carrier. Specifically, the reload cassette is insertable into the holder after removal of the existing cassette carrying the existing elongate form medicament carrier.

Optionally, the medicament dispenser is in the form of a kit of parts. Specifically, the kit comprises the body, the holder and the reload cassette, wherein the user may load the reload cassette into the holder to engage the elongate form medicament carrier with the medicament dispenser and may start the administration of the medicament dose portions thereafter.

### DETAILED DESCRIPTION OF THE DRAWINGS

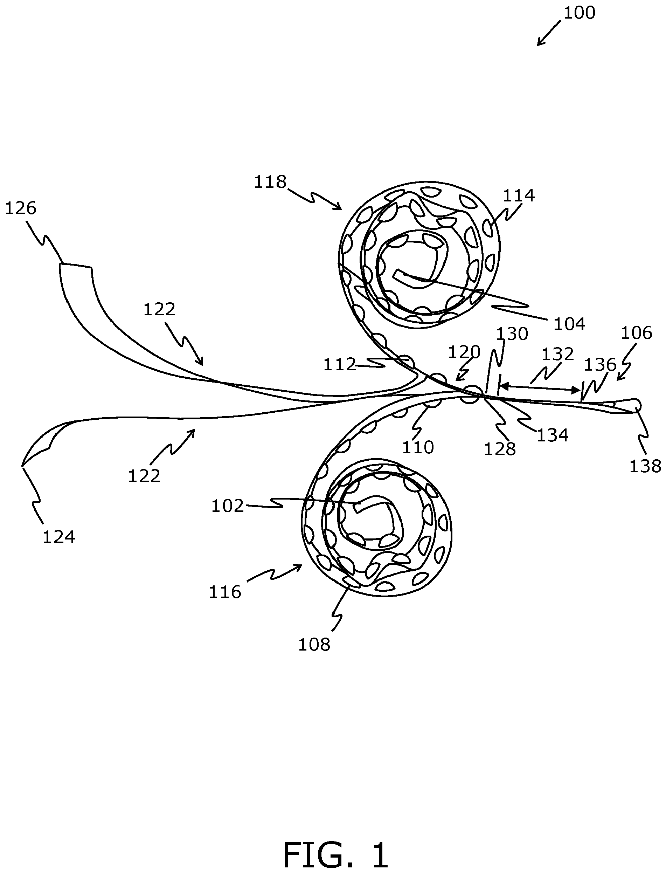

Referring to FIG. 1, illustrated is a perspective view of an elongate form medicament carrier **100,** in accordance with an embodiment of the present disclosure. The elongate form medicament carrier **100** has a first end **102,** a second end **104,** a central portion **106** extending across the width of the elongate form medicament carrier **100,** and carrying multiple distinct medicament dose portions, depicted as the exemplary medicament dose portions **108, 110, 112,** and **114.** The medicament dose portions consist of a first set of medicament dose portions, depicted as exemplary medicament dose portions **108** and **110,** and a second set of medicament dose portions, depicted as exemplary medicament dose portions **112** and **114.** The first set of medicament dose portions **108** and **110** are distributed in a first portion **116** of the elongate form medicament carrier **100** between the first end **102** and the central portion **106** of the elongate form medicament carrier **100.** The second set of medicament dose portions **112** and **114** are distributed in a second portion **118** of the elongate form medicament carrier **100** between the second end **104** and the central portion **106** of the elongate form medicament carrier **100.** As shown, the multiple distinct medicament dose portions of each of the first portion **116** and the second portion **118** of the elongate form medicament carrier **100** are uniformly spaced. Although shown with a tight bend, the central portion **106** is a continuous portion linking the first portion **116** with the second portion **118** of the elongate form medicament carrier **100.**

Moreover, the elongate form medicament carrier **100** is implemented as a combination of base elongate sheet **120** and lid elongate sheet **122.** Herein, the central portion of the lid elongate sheet **122** is divided to provide a first end region **124** and a second end region **126** of the lid elongate sheet **122.** The first end region **124** and second end region **126** are not secured to the base elongate sheet **120.**

Furthermore, the central portion **106** of the elongate form medicament carrier **100** has a first end region **128,** a second end region **130** and a unified length **132.** The unified length **132** has a trailing end **134** connecting to the first portion **116** of the elongate form medicament carrier **100** via the first end region **128** and connecting to the second portion **118** of the elongate form medicament carrier **100** via the second end region **130.** The unified length further has a leading end **136** connecting to a loop **138** of the central portion **106** of the elongate form medicament carrier **100.**

It may be understood by a person skilled in the art that FIG. 1 illustrates simplified implementations of the elongate form medicament carrier **100,** for sake of clarity, which should not unduly limit the scope of the claims herein. The person skilled in the art will recognize many variations, alternatives, and modifications of embodiments of the present disclosure.

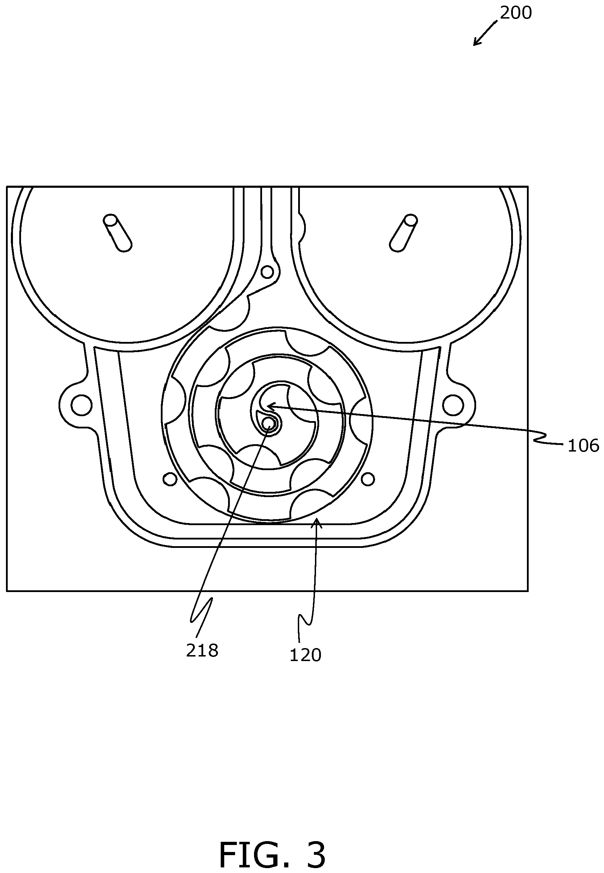

Referring to FIGs. 2 and 3, illustrated are partial sectional views of a medicament dispenser **200,** in accordance with an embodiment of the present disclosure. The medicament dispenser **200** comprises a supply of medicament active, the supply of medicament active consisting of one elongate form medicament carrier (such as the elongate form medicament carrier **100** shown in FIG. 1). The medicament dispenser **200** has a dispensing mechanism which is adapted to operate, upon each actuation of the medicament dispenser **200,** to dispense medicament active, as a single distinct medicament dose portion, from each of the first portion **116** and the second portion **118** of the elongate form medicament carrier **100.** The first portion **116** of the elongate form medicament carrier **100** contains the first set of medicament dose portions distributed between the first end **102** and the central portion **106** of the elongate form medicament carrier **100.** The second portion **118** of the elongate form medicament carrier **100** contains the second set of medicament dose portions distributed between the second end **104** and the central portion **106** of the elongate form medicament carrier **100.**

The dispensing mechanism comprises a receiving station comprising a first capstan **202** and a second capstan **204** that receives portions respectively of the first portion **116** and second portion **118** of the elongate form medicament carrier **100.** The first capstan **202** and the second capstan **204** comprise regularly distributed recesses to receive the pockets respectively of the first portion **116** and the second portion **118** of the elongate form medicament carrier **100.** The receiving station further comprises at least one indexer (not shown) that individually indexes, by means of the capstans **202** and **204,** the portions of each of the first portion **116** and the second portion **118** of the elongate form medicament carrier **100.** The dispensing mechanism further comprises a release for releasing a distinct medicament dose portion from each of the portions **116** and **118** of the elongate form medicament carrier **100.** The release comprises the arrangement of walls of the casing and manifold (discussed below) that direct the lid elongate sheet and the base elongate sheet in different directions causing them to be peeled apart, and other entities described earlier in this specification.

The receiving station further comprises a manifold **206** for dispensing the medicament active from each of the portions **116** and **118** of the elongate form medicament carrier **100.** The manifold **206** is positioned to be in communication with the distinct medicament dose portions releasable by said release to enable their dispensing to the patient.

Furthermore, the manifold **206** has a first side **208** corresponding to the first portion **116** of the elongate form medicament carrier **100** and a second side **210** corresponding to the second portion **118** of the elongate form medicament carrier **100.**

The medicament dispenser further comprises a pair of bobbins comprising a first bobbin **212** and a second bobbin **214,** the pair of bobbins configured to wind the lid elongate sheet **122** of the elongate form medicament carrier **100** thereon, starting with the first end region **124** of the lid elongate sheet **122** on the first bobbin **212** and the second end region **126** of the lid elongate sheet **122** on the second bobbin **214,** and continuing to wind first and second portions of the lid elongate sheet **122** onto the respective first bobbin **212** and second bobbin **214** after dispensing doses from the first and second sets of medicament portions respectively.

The medicament dispenser **200,** as shown in FIG. 2, further comprises a take-up spool **216** that winds up the central portion **106** of the elongate form medicament carrier **100.** Furthermore, the loop **138** of the central portion **106** of the elongate form medicament carrier **100** is secured to a peg **218** of the take-up spool **216.** As the take-up spool **216** is rotated, after winding up the central portion **106** of the elongate form medicament carrier, the take-up spool **216** continues to wind the first and second portions of the base elongate sheet **120** thereon after dispensing doses from the first and second sets of medicament portions respectively (as shown in FIG. 3). During the said process, as the first and second capstans **202** and **204** are rotated, starting with the first and second ends of the lid elongate sheet **122** the pair of bobbins **212** and **214** continue to wind the first and second portions of the lid elongate sheet **122** thereon after dispensing doses from the first and second sets of medicament portions respectively.

It may be understood by a person skilled in the art that FIGs. 2 and 3 illustrate a simplified implementation of the medicament dispenser **200,** for sake of clarity, which should not unduly limit the scope of the claims herein. The person skilled in the art will recognize many variations, alternatives, and modifications of embodiments of the present disclosure.

Referring to FIG. 4, illustrated is a schematic partial illustration of the elongate form medicament carrier **100** arranged in a medicament dispenser (as explained in FIGs. 2 and 3), in accordance with an embodiment of the present disclosure. As shown, the portions **116** and **118** of the elongate form medicament carrier **100,** containing multiple distinct medicament dose portions, depicted as the exemplary medicament dose portions **108, 110, 112,** and **114** are arranged on the first and second capstans **202** and **204** of the medicament dispenser. A pair of bobbins wind the first and second ends of the lid elongate sheet **122** as the first and second capstans **202** and **204** rotate and drive the base elongate sheet **120** after dispensing the medicament dose portions. The central portion **106** is a continuous portion linking the first portion **116** with the second portion **118** of the elongate form medicament carrier **100.** Furthermore, the central portion **106** of the elongate form medicament carrier **100** has a first end region **128,** a second end region **130** and a unified length **132.** The unified length **132** has a trailing end **134** connecting to the first portion **116** of the elongate form medicament carrier **100** via the first end region **128** and connecting to the second portion **118** of the elongate form medicament carrier **100** via the second end region **130.** The unified length further has a leading end **136** connecting to a loop **138** of the central portion **106** of the elongate form medicament carrier **100.** Furthermore, the loop **138** of the central portion **106** of the elongate form medicament carrier **100** is secured to a peg **218** of the take-up spool **216.**

Referring to FIGs. 5A and 5B, schematic arrangements **500** of a first capstan **502** and a take-up spool **504.** FIG. 5A shows a comparative arrangement and FIG.5B shows two aspects of part of an embodiment of the present disclosure, in diagrammatic form. The two aspects relate to the initial angle, **θ**, and the smaller radius of the take-up spool. The direction of rotation of the capstan and take up spool is shown by curved hollow arrows in each case. Herein, '|' corresponds to a distance between the capstan **502** and the edge of the take-up spool **504,** at a point marked A, facing the capstan **502** along a tangent from the capstan **502;** 'r' and 's' correspond to radii of the capstans **502** and the take-up spool **504,** respectively; although, in FIG. 5A r=s. In FIG. 5A, 'm' corresponds to an ideal length of unwound elongate form medicament carrier after the take-up spool **504** and the capstan **502** have rotated a quarter turn, i.e. by 90 degrees. As shown in FIG. 5B, the radius of the take-up spool **504** is smaller than the first capstan **502.** 'm' corresponds to an ideal length of unwound elongate form medicament carrier when the take-up spool **504** has reached the 90-degree position. For convenience, the smaller radius of the take-up spool **504** is shown along with the angle **θ**, which corresponds to the starting position A' of the peg on the take-up spool when the elongate form medicament carrier **100** is installed into the dispenser. In FIG. 5A, **A'** is at the 12 o'clock position; whereas in FIG. 5B, **A'** is indicated at the 11 o'clock position. The distance between the capstan and the changed initial position of the peg is indicated as k. During rotation of the capstan indicated as arc B, the peg rotates along an arc C.

Modifications to embodiments of the present disclosure described in the foregoing are possible without departing from the scope of the present disclosure as defined by the accompanying claims. Expressions such as "including", "comprising", "incorporating", "have", "is" used to describe and claim the present disclosure are intended to be construed in a non-exclusive manner, namely allowing for items, components or elements not explicitly described also to be present. Reference to the singular is also to be construed to relate to the plural.

## Claims

1. An elongate form medicament carrier carrying multiple distinct medicament dose portions and having a first end, a second end, a central portion extending across the width of the elongate form medicament carrier, a first portion of the elongate form medicament carrier between the first end and the central portion and containing a medicament active, or a mixture of medicament actives, and a second portion of the elongate form medicament carrier between the second end and the central portion and containing a medicament active, or a mixture of medicament actives;
wherein the central portion of the elongate form medicament carrier has a first end region, a unified length and a second end region, the unified length has a trailing end connecting to the first portion of the elongate form medicament carrier via the first end region and connecting to the second portion of the elongate form medicament carrier via the second end region, and the unified length has a leading end connecting to a loop of the central portion of the elongate form medicament carrier.

2. An elongate form medicament carrier according to claim 1, in which the unified length is a bonded portion formed from two lengths of elongate form medicament carrier bonded together face-to-face.

3. An elongate form medicament carrier according to claim 1 or claim 2, in which the elongate form medicament carrier comprises a lid elongate sheet substantially secured to a base elongate sheet having pockets containing the multiple distinct medicament dose portions, and the base elongate sheet comprises the unified length of the central portion of the elongate form medicament carrier.

4. An elongate form medicament carrier according to claim 3, the lid elongate sheet having a central portion corresponding to the central portion of the elongate form medicament carrier, wherein the central portion of the lid elongate sheet is divided to provide a first inner end and a second inner end of the lid elongate sheet, and the first inner end and second inner end are not secured to the base elongate sheet.

5. An elongate form medicament carrier according to any one of the preceding claims, in which the medicament dose portions consist of a first set and a second set of medicament dose portions, the first set of medicament dose portions distributed in the first portion of the elongate form medicament carrier and the second set of medicament dose portions distributed in the second portion of the elongate form medicament carrier.

6. An elongate form medicament carrier according to claim 5 wherein the dose portions in the second set of medicament dose portions are different from the dose portions in the first set of medicament dose portions.

7. An elongate form medicament carrier according to claim 6 wherein the dose portions in the second set of medicament dose portions differ from the dose portions in the first set of medicament dose portions in that the dose portions in the second set of medicament dose portions contain a medicament active, or a mixture of medicament actives that is different from that in the first set of medicament dose portions.

8. An elongate form medicament carrier according to any one of the preceding claims wherein the length of the unified length is in the range 5 millimetres to 30 millimetres.

9. A medicament dispenser comprising a supply of medicament active, the supply of medicament active consisting of one elongate form medicament carrier according to any one of the preceding claims, said dispenser having a dispensing mechanism which is adapted to operate, upon each actuation of the medicament dispenser, to dispense medicament active from each of the first and second portions of the elongate form medicament carrier, said mechanism comprising,
a receiving station comprising first and second capstans that receive portions respectively of the first and second portions of the elongate form medicament carrier and at least one indexer that individually indexes the portions of each of the first and second portions of the elongate form medicament carrier,
the receiving station further comprising a manifold, for dispensing the medicament active from each of the first and second portions of the elongate form medicament carrier,
the manifold having a first side corresponding to the first portion of the elongate form medicament carrier and a second side corresponding to the second portion of the elongate form medicament carrier,
the medicament dispenser further comprising a take-up spool that winds up the central portion of the elongate form medicament carrier;
wherein the loop of the central portion of the elongate form medicament carrier is secured to a peg of the take-up spool.

10. A medicament dispenser according to claim 9, the take-up spool having an axis of rotation such that the edge of the take-up spool facing the manifold rotates towards the first side of the manifold; wherein the peg is positioned away from the axis of rotation and in a rotationally advanced position, relative to a datum line from the point where the elongate form medicament carrier leaves the first capstan to the axis of rotation of the take-up spool, prior to the indexer being driven for the first time after assembly of the elongate form medicament carrier into the medicament dispenser.

11. A medicament dispenser according to claim 10, wherein the peg is in a position rotationally advanced at least 30 degrees relative to the datum line.

12. A medicament dispenser according to claim 11, wherein the peg is in a position rotationally advanced at least 60 degrees relative to the datum line.

13. A medicament dispenser according to any one of claims 10 to 12, wherein the peg is in a position rotationally advanced not more than 90 degrees relative to the datum line.

14. A medicament dispenser according to any one of claims 9 to 13, wherein the radius of the take-up spool, where the central portion of the elongate form medicament carrier is initially wound on, is less than the radii of the first and second capstans at the respective points where the elongate form medicament carrier leaves the first and second capstans.

15. A medicament dispenser according to claim 14, wherein the indexing mechanism is configured to drive the capstans and the take-up spool with gears such that their respective gear ratios are in inverse proportion to their respective radii.
